(19) Europäisches Patentamt
European Patent Office
Office européen des brevets

(11) Numéro de publication : **0 443 899 A1**

(12) **DEMANDE DE BREVET EUROPEEN**

(21) Numéro de dépôt : **91400299.3**

(51) Int. Cl.$^5$ : **H01B 3/22,** C07C 15/16

(22) Date de dépôt : **07.02.91**

(30) Priorité : **20.02.90 FR 9002013**

(43) Date de publication de la demande :
**28.08.91 Bulletin 91/35**

(84) Etats contractants désignés :
**AT BE CH DE DK ES FR GB GR IT LI LU NL SE**

(71) Demandeur : **ATOCHEM**
**4 & 8, Cours Michelet La Défense 10**
**F-92800 Puteaux (FR)**

(72) Inventeur : **Commandeur, Raymond**
**Le Rocher, Avenue de Vénaria**
**F-38220 Vizille (FR)**
Inventeur : **Berger, Noelle**
**Frênes 4, Charrière Blanche**
**F-69130 Ecully (FR)**
Inventeur : **Jay, Pierre**
**89 Rocade des Monts d'Or**
**F-69370 Saint-Didier au Mont d'Or (FR)**

(54) **Procédé de synthèse d'oligomères de (méthylbenzyl)xylène et leur application comme diélectrique.**

(57) L'invention concerne un procédé de synthèse d'oligomères de (méthylbenzyl)xylène.
Elle concerne aussi un nouveau liquide diélectrique constitué d'un mélange de (méthylbenzyl)xylène et de di(méthylbenzyl)xylène.

EP 0 443 899 A1

# PROCEDE DE SYNTHESE D'OLIGOMERES DE (METHYLBENZYL)XYLENE ET LEUR APPLICATION COMME DIELECTRIQUE

La présente invention concerne un procédé de synthèse d'oligomères de (méthylbenzyl)xylène.

On a décrit dans la demande de brevet EP 299 867 des oligomères de polyarylalcanes qui peuvent être préparés par chloration du toluène ou du xylène suivie d'une condensation sur le xylène par une réaction de FRIEDEL et CRAFTS. La demanderesse a découvert que certains de ces produits étaient des diélectriques.

La présente invention est donc l'application comme fluide diélectrique des oligomères A ou B suivants ou du mélange de A et B.

L'oligomère A, qu'on appelle oligomère du (méthylbenzyl)xylène est un isomère ou un mélange d'isomères de formule :

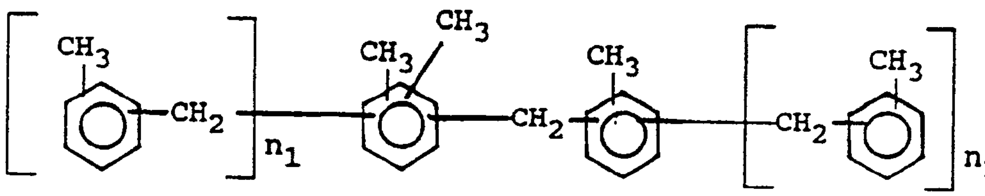

(A)

dans laquelle $n_1$ et $n_2$ = 0, 1 ou 2 sachant que $n_1 + n_2$ est inférieur ou égal à 3.

L'oligomère B est un isomère ou un mélange d'isomères de formule :

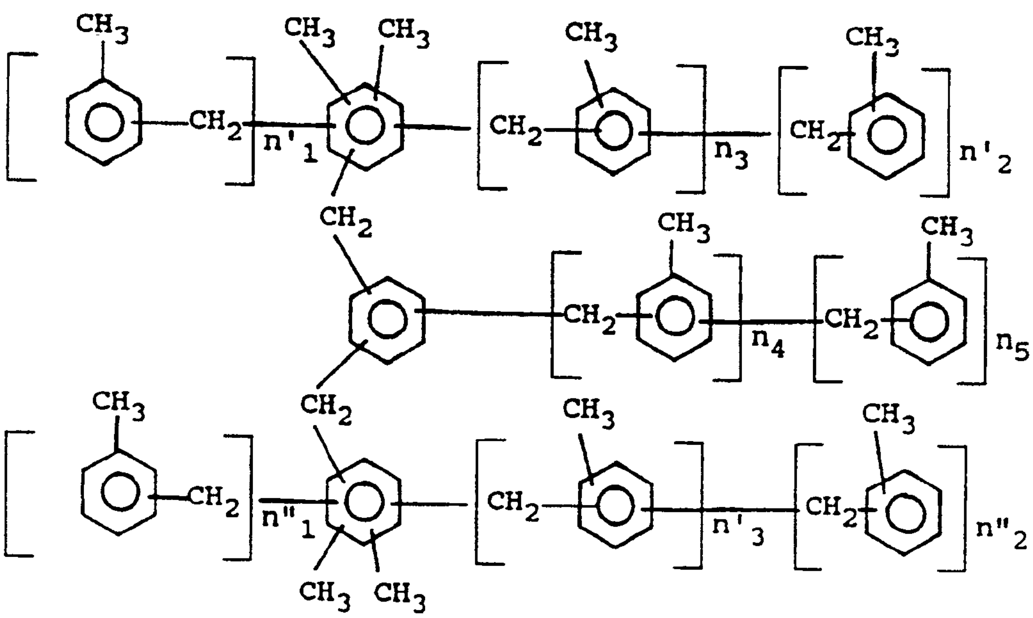

dans laquelle $n'_1$, $n''_1$ et $n_4$ valent 0, 1 ou 2

$n'_2$, $n''_2$, $n_3$, $n'_3$ et $n_5$ valent 0 ou 1

sachant que $n'_1 + n''_1 + n'_2 + n''_2 + n_3 + n'_3 + n_4 + n_5$, qu'on désigne par Sn est inférieur ou égal à 2.

Parmi les oligomères du (méthylbenzyl)xylène A, on appelle (méthylbenzyl)xylène ceux dans lesquels $n_1 + n_2 = 0$ et di(méthylbenzyl)xylène ceux dans lesquels $n_1 + n_2 = 1$.

Ces oligomères s'utilisent comme diélectriques dans les transformateurs, les condensateurs, ou même dans les câbles électriques. Pour les applications diélectriques, il est recommandé de purifier les produits par exemple à l'aide de terres décolorantes, d'adsorbants et de les stabiliser par addition d'époxydes. Ces traitements de purification et les différents additifs sont connus en eux-mêmes.

On ne sortirait pas du cadre de l'invention en utilisant les oligomères A ou B ou A et B en mélange avec d'autres fluides diélectriques.

La présente invention concerne aussi, à titre de nouveau produit, un mélange d'oligomères A contenant des produits tels que $n_1 + n_2 = 0$ et $n_1 + n_2 = 1$, c'est-à-dire du (méthylbenzyl)xylène et du di(méthylbenzyl)xylène .

La demanderesse a découvert de façon inattendue que, bien que le (méthylbenzyl)xylène préparé à partir de l'orthoxylène cristallise à -40°C et que le di(méthylbenzyl)xylène préparé à partir de l'orthoxylène soit solide à la température ambiante, leur mélange cristallise à plus basse température. En particulier un mélange contenant 5 à 30 parties de di(méthylbenzyl)xylène pour respectivement 95 à 70 parties de (méthylbenzyl)xylène cristallise entre -50 et -60°C. On ne sortirait pas du cadre de l'invention si le mélange précédent des oligomères A, c'est-à-dire le mélange des oligomères tels que $n_1 + n_2 = 0$ et $n_1 + n_2 = 1$ contenait aussi des oligomères B.

A titre d'exemples, les mélanges tels que :

| | | | | |
|---|---|---|---|---|
| $n_1 + n_2 = 0$ | 85 | 85 | 80 | 80 |
| $n_1 + n_2 = 1$ | 15 | 10 | 20 | 20 |
| Sn = 0 | 2 | 4 | 2 | 4 |

présentent une cristallisation inférieure à -40°C.

L'oligomère A peut être préparé, par exemple, par condensation du chlorure de méthylbenzyle sur le xylène. L'oligomère B peut se préparer par condensation du dichlorure de xylène $CH_2Cl-C_6H_4-CH_2Cl$ soit sur l'oligomère A et éventuellement du chlorure de méthylbenzyl, soit sur un mélange de xylène et de chlorure de méthylbenzyl.

La présente invention concerne aussi un procédé de synthèse d'oligomères du (méthylbenzyl)xylène qui est caractérisé en ce qu'on effectue la condensation du chlorure de méthylbenzyle $CH_3-C_6H_4-CH_2Cl$ sur le xylène ou sur des oligomères inférieurs du (méthylbenzyl)xylène, en présence d'un catalyseur de FRIEDEL et CRAFTS.

Les oligomères inférieurs du (méthylbenzyl)xylène sont semblables aux oligomères du (méthylbenzyl)xylène, mais contiennent moins de motifs méthylbenzyl. Par exemple le (méthylbenzyl)xylène ($n_1 + n_2 = 0$) est un oligomère inférieur du (méthylbenzyl)xylène par comparaison avec le di(méthylbenzyl)xylène ($n_1 + n_2 = 1$).

On ne sortirait pas du cadre de l'invention si la condensation du chlorure de méthylbenzyle s'effectue sur un mélange de xylène et d'oligomères inférieurs du (méthylbenzyl)xylène. La condensation en présence d'un catalyseur de FRIEDEL et CRAFTS est une réaction connue en soi. Elle a lieu en pratique à une température comprise entre 50 et 130°C. La quantité de catalyseur est habituellement comprise entre 50 ppm et 1 % en poids de la masse réactionnelle. A titre d'exemple, des catalyseurs de FRIEDEL et CRAFTS ont été décrits dans le brevet européen EP 136 230. Il est recommandé après distillation du xylène en excès de procéder à l'élimination du catalyseur par toute technique connue telle que lavage à l'eau, neutralisation, séchage.

Il suffit ensuite d'une simple distillation pour séparer les différents oligomères de (méthylbenzyl)xylène. Le mélange réactionnel qu'on vient d'obtenir après la condensation et qui est constitué essentiellement d'oligomères du (méthylbenzyl)xylène et d'un excès éventuel de xylène peut. contenir des produits chlorés organiques tels que :

– des xylène chlorés par exemple :

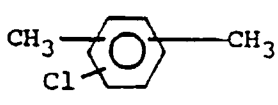

– du (méthylchlorobenzyl)xylène :

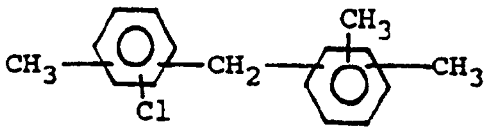

et d'une façon générale des oligomères de (méthylbenzyl)xylène portant un ou plusieurs atomes de chlore sur le noyau benzénique. Ces produits ont été apportés ou ont été formés à partir des impuretés du chlorure de méthylbenzyle ou éventuellement par le catalyseur de FRIEDEL et CRAFTS. Pour certaines applications, il est souhaitable que les oligomères de (méthylbenzyl)xylène contiennent très peu ou pas du tout de chlore.

Selon une forme particulière de l'invention, on traite les oligomères de (méthylbenzyl)xylène pour éliminer les produits chlorés organiques. Il est recommandé d'effectuer le traitement après l'élimination du xylène en excès et du catalyseur.

Après la réaction de condensation on élimine donc le xylène en excès, puis le catalyseur et sur ce mélange brut d'oligomères du (méthylbenzyl)xylène on effectue une élimination du chlore organique.

On peut utiliser tout procédé de destruction des produits chlorés organiques, par exemple le procédé décrit dans EP 306 398 utilisant un alcoolate de métal alcalin, le procédé décrit dans EP 250 748 utilisant un alcoolate lourd, le contenu de ces demandes étant incorporé dans la présente invention. On préfère utiliser le procédé de déchloration décrit dans EP 306 398.

Selon le procédé préféré, on met en contact le mélange brut avec un alcoolate et on porte l'ensemble sous agitation à une température comprise entre 220 et 320°C. L'alcoolate est de préférence un alcoolate de sodium, par exemple du méthylate de sodium.

Après le traitement de déchloration, il suffit d'une simple distillation pour récupérer les oligomères de (méthylbenzyl)xylène à faible teneur en chlore. On obtient en pied une fraction lourde contenant les restes de l'agent déchlorant, un chlorure alcalin (NaCl) et des oligomères lourds de (méthylbenzyl)xylène.

On ne sortirait pas du cadre de l'invention si on recyclait, en partie ou en totalité, cette fraction lourde obtenue en pied et qu'on l'utilisait seule ou en mélange avec le produit mis en oeuvre pour détruire les produits chlorés organiques.

On ne sortirait pas du cadre de l'invention si après la condensation et après élimination du xylène, on recyclait à l'étape de condensation, en tout ou partie, le (méthylbenzyl)xylène. L'avantage de ce recyclage est d'augmenter la proportion de di(méthylbenzyl)xylène dans les oligomères. Le xylène éliminé peut être réutilisé en amont dans le procédé. On pourrait aussi prendre tout ou partie du (méthylbenzyl)xylène après la déchloration et le recycler à l'étape de condensation.

Le chlorure de méthylbenzyle peut contenir aussi du dichlorure de xylène ($CH_2Cl$-$C_6H_4$-$CH_2Cl$). On obtient alors, en mélange avec les oligomères du (méthylbenzyl)xylène (A), des oligomères B.

On ne sortirait pas du cadre de l'invention si on effectuait la chloration radicalaire du xylène puis qu'on procédait directement sur ce mélange à la condensation. Il suffit après la chloration d'ajouter le catalyseur de FRIEDEL et CRAFTS dans ledit mélange.

Il va de soi que le procédé décrit peut être mis en oeuvre en continu ou en discontinu.

La demanderesse a découvert aussi que si on effectue le procédé de l'invention avec du chlorure ferrique comme catalyseur de FRIEDEL et CRAFTS, alors il n'est pas nécessaire d'éliminer le catalyseur, c'est-à-dire qu'on peut distiller le mélange obtenu après la réaction de condensation pour obtenir, d'abord le xylène, puis les oligomères de (méthylbenzyl)xylène avec éventuellement les oligomères B ; tous ces oligomères étant aptes à l'usage diélectrique. Cette propriété n'est pas vraie pour le chlorure d'aluminium, si on n'élimine pas le chlorure d'aluminium après la condensation, très souvent on ne peut même pas distiller le mélange d'oligomères. Si on arrive à récupérer des oligomères de (méthylbenzyl)xylène, alors ils sont inaptes à l'usage diélectrique. La demanderesse a aussi découvert que le fait d'utiliser le chlorure ferrique comme catalyseur de condensation permet non seulement d'éviter son traitement d'élimination, mais qu'on peut procéder dès la fin de la condensation à la destruction éventuelle des produits chlorés organiques comme on l'a décrit précédemment. Il est quand même recommandé d'éliminer le xylène en excès dès la fin de réaction de condensation.

Après le traitement de déchloration, il suffit d'une simple distillation pour récupérer les oligomères du (méthylbenzyl)xylène et éventuellement les oligomères B à faible teneur en chlore. On obtient en pied une fraction lourde contenant les restes de l'agent déchlorant, du chlorure alcalin, des sels de fer et des oligomères lourds. Comme précédemment cette fraction lourde peut être recyclée et servir d'appoint à l'agent déchlorant.

## EXEMPLE 1

Dans un réacteur muni d'une agitation, d'un condenseur, d'un tube d'alimentation de chlore et d'une lampe PHILIPS TLADK de 30 Watt, on place 424 g d'orthoxylène (4 moles) ; on introduit ensuite 71 g de chlore gazeux (1 mole) en maintenant la température à 80°C durant 1 heure.

Après arrêt de l'initiation photochimique, le milieu réactionnel est placé dans une ampoule de coulage et il est introduit en 1 heure dans un réacteur muni d'une agitation contenant 2 moles d'orthoxylène et 60 mg de $FeCl_3$, à une température de 100°C. L'ensemble est maintenu encore 1 heure à 100°C sous agitation après fin de coulage. L'excès d'orthoxylène est éliminé par distillation sous vide de 10 mm de mercure avec une colonne de quelques plateaux de manière à ce que la teneur résiduelle en orthoxylène dans le produit en pied soit inférieure à 500 ppm (température de pied en fin de distillation = 190°C).

Nous obtenons un mélange d'oligomères de polyarylalcanes de type A et B avec du chlorure de fer en suspension. La composition de la partie organique est la suivante :

| | $n_1 + n_2$ | | | | $S_n$ | | |
|---|---|---|---|---|---|---|---|
| **PRODUIT** | 0 | 1 | 2 | 3 | 0 | 1 | 2 |
| A | 73 | 14,6 | 3,7 | 1,7 | | | |
| B | | | | | 5,4 | 1,3 | 0,3 |

Le rendement pondéral calculé d'après l'orthoxylène consommé est de 97 %.

Ce mélange est ensuite soumis à un traitement par 2 % de méthylate de sodium 6 Heures à 300°C sous couverture d'azote. Le mélange issu de ce traitement est soumis à une distillation avec quelques plateaux sous 0,5 mm de mercure. Nous obtenons :

1°) une fraction d'un liquide incolore distillant à la température de 120-140°C constituée par le produit A $n_1 + n_2 = 0$. Ce produit sera dénomé XX01 (méthylbenzyl)xylène.

2°) une fraction d'un liquide jaune clair visqueux distillant à la température de 195-215°C cristallisant lentement à température ambiante. La composition en poids est la suivante :

75 % de composé A $n_1 + n_2 = 1$ di(méthylbenzyl)xylène

25 % de composé B $S_n = 0$

Cette fraction sera dénomée XX02.

La teneur en chlore dans chacune des fractions est inférieure à 5 ppm.

EXEMPLE 2

Nous avons comparé différents liquides par la mesure de leur tension de claquage en courant alternatif 50 Hz dans les 2 conditions suivantes (méthodes 1 et 2) :

1 - rampe 2000 V/s - électrodes dissymétriques.
- pointe diamètre 0,6 mm
- disque à bords arrondis Rogowski, diamètre 39,6 mm
- distance interélectrode = 3,2 mm

2 - rampe 1000 V/30s - électrodes dissymétriques
- pointe diamètre 0,6 mm
- disque diamètre 39,6 mm
- distance 3,2-10-15-20 mm

Les résultats sont reportés dans le tableau ci-après :

| METHODE | | | | LIQUIDE | | | |
|---|---|---|---|---|---|---|---|
| n° | rampe de tension | Electrode | d inter. mm | XX01 | PXE | DDB | Huile Minérale |
| 1 | 2000V/s | pointe Ø= 0,6<br>disque Ø=39,6 | 3,2 | 53,0 *<br>(48,6-57,2)**<br>(5) *** | 47,9<br>(43,5-52)<br>(10) | 39,0<br>(34,9-44,4)<br>(10) | 29,9<br>(27,3-34,9)<br>(5) |
| 2 | 1000V toutes les 30 sec. | pointe Ø= 0,6<br>disque Ø=39,6 | 3,2 | 41,2<br>(37,7-45,3)<br>(5) | 37,8<br>(34-39,7)<br>(5) | | 25,4<br>(21,5-28,5)<br>(10) |
| | | | 10 | 69,5<br>(65,3-75,8)<br>(5) | 53,2<br>(48,2-58,7)<br>(6) | | 34,3<br>(28-41,5)<br>(10) |
| | | | 15 | 82,4<br>(74-89)<br>(5) | 75,8<br>(69,0-84,5)<br>(5) | | 35,6<br>(30-38)<br>(10) |
| | | | 20 | 93,8<br>(90,0-105,0)<br>(5) | 74,7<br>(71,0-76,5)<br>(5) | | 45,6<br>(39,5-56,0)<br>(10) |

* La première ligne indique la moyenne, ** La deuxième ligne indique les valeurs extrêmes, *** La troisième ligne indique le nombre des mesures

XX01 désigne un produit de l'exemple 1, PXE désigne un produit vendu sous le nom de NISSEKI CONDENSOR OIL S qui est un mélange de phényl 1-xylyl 1-éthane et de phényl-1 éthylphényl 1-éthane.
DDB désinge du dodécylbenzène ramifié, l'huile minérale est une huile du commerce pour transformateurs.

EXEMPLE 3

Cristallisation du produit XXO1 de l'exemple 1, du produit XX02 de l'exemple 1, et d'un mélange de 85 % en poids de XXO1 et 15 % en poids de XX02 (voir exemple 1) qu'on appelle XX05.
Le XX05 est donc constitué de :
- 85 parties de $A/n_1 + n_2 = 0$
- 15 parties de XXO2 à 75 % de $A/n_1 + n_2 = 1$ et 25 % de $B/Sn = 0$, c'est-à-dire :
  15 x 0,75 = 11,25 parties de $A/n_1 + n_2 = 1$
  15 X 0,25 = 3,75 parties de $B/Sn = 0$

Le XX01 cristallise à -40°C, le XX02 est cristallisé à température ambiante et le XX05 n'est pas encore cristallisé à -50°C. Au bout de quelques mois il apparaît un début de cristallisation sur certains échantillons de XX05.

EXEMPLE 4 :

On effectue les mêmes essais que dans l'exemple 3 mais avec des produits XX01 et XX02 préparés comme dans l'exemple 1 sauf qu'on utilise un mélange de 75 % d'orthoxylène et 25 % de paraxylène ou bien de 100 % d'orthoxylène.
Aucun des échantillons de XX05 ne présente de début de cristallisation même après plusieurs mois.

**Revendications**

1. Application comme fluide diélectrique des oligomères A ou B suivants ou du mélange de A et B, l'oligomère A étant un mélange d'isomères de formule :

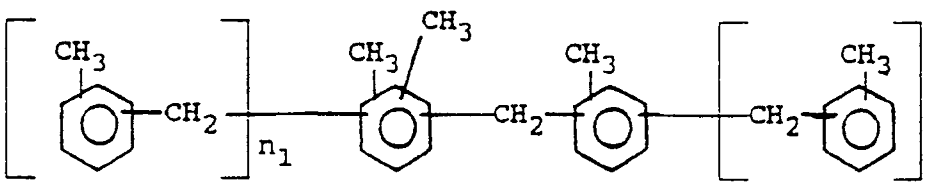
(A)

dans laquelle $n_1$et $n_2$ = 0, 1 et 2 sachant que $n_1 + n_2$ est inférieur ou égal à 3.
L'oligomère B est un mélange d'isomères de formule :

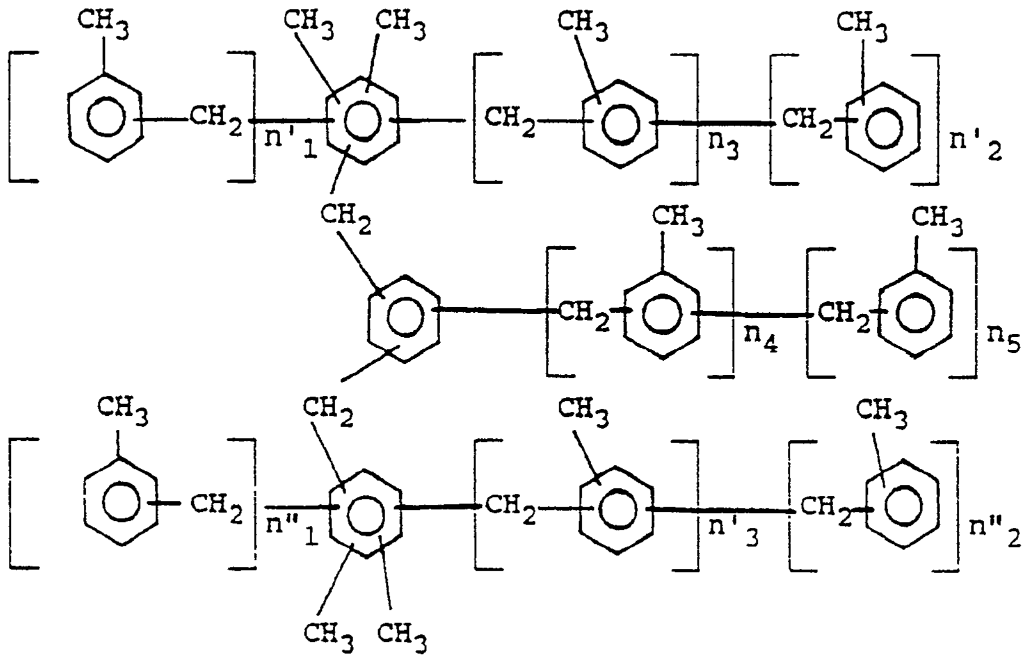

dans laquelle $n'_1$, $n''_1$ et $n_4$ valent 0, 1 et 2
$n'_2$, $n''_2$, $n_3$, $n'_3$ et $n_5$ valent 0 et 1
sachant que $n'_1 + n''_1 + n'_2 + n''_2 + n_3 + n'_3 + n_4 + n_5$, qu'on désigne par Sn est inférieur ou égal à 2.

2. Composition à base d'oligomères du (méthylbenzyl)xylène de formule :

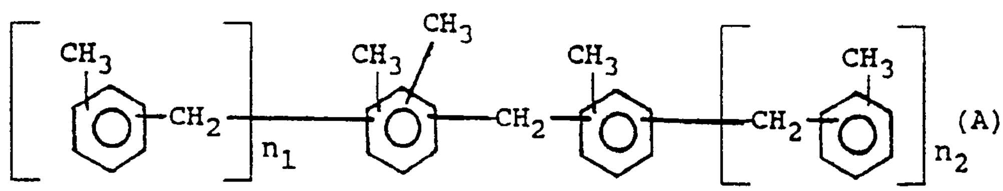

(A)

dans laquelle $n_1$ et $n_2$ = 0, 1 et 2, sachant que $n_1 + n_2$ est inférieur ou égal à 3, caractérisée en ce qu'elle comprend des oligomères tels que $n_1 + n_2 = 0$ et des oligomères tels que $n_1 + n_2 = 1$.

3. Composition selon la revendication 2, caractérisée en ce que le rapport en poids des oligomères tels que $n_1 + n_2 = 0$ et des oligomères tels que $n_1 + n_2 = 1$ est de 95/5 à 70/30.

4. Composition selon la revendication 3, caractérisée en ce que les proportions précédentes sont de préférence 85/10 à 80/20.

5. Procédé de synthèse d'oligomères du (méthylbenzyl)xylène, caractérisé en ce qu'on effectue la condensation du chlorure de méthylbenzyle sur le xylène ou sur des oligomères inférieurs du (méthylbenzyl)xylène en présence d'un catalyseur de FRIEDEL et CRAFTS.

6. Procédé selon la revendication 5, caractérisé en ce qu'après la condensation on élimine les produits chlorés organiques par réaction avec du sodium ou un alcoolate de métal alcalin et de préférence du méthylate de sodium.

7. Procédé selon la revendication 5 ou 6, caractérisé en ce qu'on recycle en tout ou partie le (méthylbenzyl)xylène à l'étape de condensation.

8. Procédé selon l'une des revendications 6 à 7, caractérisé en ce qu'on recycle en partie ou en totalité, à

l'étape d'élimination des produits chlorés organiques, la fraction lourde qu'on obtient dans la distillation pour récupérer les oligomères de (méthylbenzyl)xylène à faible teneur en chlore.

**9.** Procédé selon l'une des revendications 5 à 8, caractérisé en ce qu'on utilise le chlorure ferrique comme catalyseur de condensation.

**10.** Procédé selon la revendication 9, caractérisé en ce qu'on effectue l'élimination des produits chlorés organiques sans avoir éliminé le chlorure ferrique.

**11.** Application selon la revendication 1, notamment pour les condensateurs, les transformateurs et les cables.

Office européen des brevets

# RAPPORT DE RECHERCHE EUROPEENNE

Numero de la demande

EP 91 40 0299

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| D,X | EP-A-0 136 230 (ATOCHEM)<br>* Revendications; page 2, lignes 1-11 *<br>--- | 1-11 | H 01 B 3/22<br>C 07 C 15/16 |
| X | EP-A-0 299 866 (ATOCHEM)<br>* Revendications; page 2, lignes 5-15 *<br>--- | 1-11 | |
| D,X | EP-A-0 299 867 (ATOCHEM)<br>* Revendications; page 2, lignes 5-15 *<br>----- | 1-11 | |
| | | | DOMAINES TECHNIQUES RECHERCHES (Int. Cl.5) |
| | | | H 01 B<br>C 07 C |

Le présent rapport a été établi pour toutes les revendications

| Lieu de la recherche | Date d'achèvement de la recherche | Examinateur |
|---|---|---|
| LA HAYE | 12-04-1991 | LEROY ALAIN |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)